(19) **Europäisches Patentamt / European Patent Office / Office européen des brevets**

(11) **EP 2 323 485 B1**

(12) **EUROPEAN PATENT SPECIFICATION**

(45) Date of publication and mention
of the grant of the patent:
**16.10.2013 Bulletin 2013/42**

(21) Application number: **09781601.1**

(22) Date of filing: **07.08.2009**

(51) Int Cl.:
*A61K 31/28* (2006.01)    *A61K 33/06* (2006.01)
*A61K 33/10* (2006.01)    *A61K 33/24* (2006.01)
*A61K 33/42* (2006.01)    *A61K 45/06* (2006.01)
*A01N 59/06* (2006.01)    *A01N 59/08* (2006.01)

(86) International application number:
**PCT/EP2009/060260**

(87) International publication number:
**WO 2010/015697 (11.02.2010 Gazette 2010/06)**

(54) **ANTI-ALLERGEN COMBINATIONS OF CALCIUM AND LANTHANUM SALTS**

ANTIALLERGENE KOMBINATIONEN AUS KALZIUM- UND LANTHANSALZEN

COMBINAISONS ANTI-ALLERGÉNIQUES DE CALCIUM ET DE SELS DE LANTHANE

(84) Designated Contracting States:
**AT BE BG CH CY CZ DE DK EE ES FI FR GB GR HR HU IE IS IT LI LT LU LV MC MK MT NL NO PL PT RO SE SI SK SM TR**
Designated Extension States:
**AL BA RS**

(30) Priority: **08.08.2008 EP 08162069**

(43) Date of publication of application:
**25.05.2011 Bulletin 2011/21**

(73) Proprietor: **Janssen Pharmaceutica, N.V.**
**2340 Beerse (BE)**

(72) Inventor: **BYLEMANS, Dany, Leopold, Jozefien**
**B-2340 Beerse (BE)**

(74) Representative: **Verberckmoes, Filip Gerard**
**Janssen Pharmaceutica N.V.**
**J&J Patent Law Department**
**Turnhoutseweg 30**
**2340 Beerse (BE)**

(56) References cited:
**EP-A- 1 131 999    EP-A- 1 133 918**
**DD-A1- 280 692**

- **MARTYNY ET AL: "Aerosolized sodium hypochlorite inhibits viability and allergenicity of mold on building materials" JOURNAL OF ALLERGY AND CLINICAL IMMUNOLOGY, MOSBY - YEARLY BOOK, INC, US, vol. 116, no. 3, 1 September 2005 (2005-09-01), pages 630-635, XP005068195 ISSN: 0091-6749**
- **JERRIM K L ET AL: "Electrostatic enhancement of dust and allergen removal from carpets" JOURNAL OF ELECTROSTATICS, ELSEVIER SCIENCE PUBLISHERS B.V. AMSTERDAM, NL, vol. 53, no. 1, 1 June 2001 (2001-06-01), pages 39-51, XP004246742 ISSN: 0304-3886**

**Description**

The present invention relates to certain combinations of alkaline earth metal salts and rare earth metal salts which provide an improved denaturing effect on allergens. More particularly, the present invention relates to compositions comprising a combination of calcium salts and lanthanum salts in respective proportions to produce a synergistic denaturing effect on allergens. Compositions comprising these combinations are particularly effective for use in an *ex vivo* method for denaturing allergens associated with house dust mites and other common allergens such as cat dander, molds, cockroach, pollen and the like.

A lot of people suffer from allergic reactions. These allergic reactions can be caused by exposure to allergens originating from house dust mites that live in the house, saliva or hair of pets, molds, insects and various kinds of pollen. Allergic reactions can be initiated in many ways, but one of the most common ways is by the inhalation of airborne allergens. Another common way to ingest allergens is when they come in direct contact with a moist surface on the body where they stick and react with the surrounding body tissue. This can happen, e.g., when a person lays down in bed and allergen containing dust that lay on the pillow or bed coverings contacts and is absorbed into the person's eyes. This typically results in an allergic reaction as the allergens are gradually absorbed into the mucous around the eye. Other symptoms related to allergen exposure are allergic rhinitis, rhinoconjunctivitis, eczematous dermatitis, pulmonary inflammation, wheezing, bronchial asthma or even life threatening systemic anaphylaxis. Allergic people in contact with allergens suffer from sneezing, nasal congestion, watery eyes, fatigue, itching, difficulties in breathing with as secondary consequences tiredness, drowsiness and generally a decreased quality of life.

It is believed that allergens in the faeces of the house dust mite, *Dermatophagoides farinae*, (known as Der-f) and *Dermatophagoides pteronyssinus* (known as Der-p) trigger the immune response of the body, thereby giving rise to the well known allergic reactions. Other allergens which are problematic are cat allergens (known as Fel-d), dog allergens (Can-f), plant pollen allergens like from trees (like Bet-v) or grass (like Phl-p), rodents (Mus-m, Rat-n), storage mites (like Tyr-p or Blo-t), molds (like Alt-a and Asp-f) and cockroach allergens (known as Bla-g).

A method to avoid allergic reactions upon exposure to allergens is the removal of these allergens from the environment where allergic people live. Allergens can be removed from the environment where allergic people live using a vacuum cleaner or an air conditioner. However, a vacuum cleaner cannot remove all of the allergens that exist in the house, and an air conditioner can only remove airborne allergens. Another method to protect allergic people against exposure to allergens is by using high-density covers on the bedclothes. However, these covers merely protect people from allergens inside of bedclothes and is not useful for the allergens that come from the environment. None of these methods are fully satisfactory.

Generally acaricides are used for controlling house dust mites. However, house dust mites, such as *Dermatophagoides farinae*, *Dermatophagoides pteronyssinus*, can also be the source of allergens even after their death as the dead bodies of house dust mites gradually decompose and release fine particles of allergens. As a result, controlling of house dust mites by applying acaricides is not always sufficient to remove allergens from the environment.

Recently some methods for chemically denaturing allergens have been developed. For example, methods that use tannic acid and extracts of tea leaf, gallic acid and so on are proposed. However, it is hard to get steady effectiveness for denaturing allergens using these proposed methods. Furthermore, these methods have the disadvantage of causing coloring on the materials treated with the proposed denaturing agent.

EP- 1, 133, 918 discloses a method for denaturing allergens which comprises applying an effective amount of an alkaline earth metal salt selected from calcium and strontium salts to the place where allergens exist or will exist.

EP- 1, 131, 999 discloses a method for denaturing allergens which comprises applying an effective amount of a rare earth metal salt to the place where allergens exist.

It has now been found that the combination of calcium salts and lanthanum salts has a synergistic denaturating effect on allergens.

In a first embodiment the present invention relates to a composition comprising a combination of one or more calcium salts as component (I) and one or more lanthanum salts as component (II) wherein component (I) and component (II) are in respective proportions to produce a synergistic denaturing effect on allergens.

Typical examples of the calcium salt include calcium acetate, calcium propionate, calcium nitrate, calcium chloride, calcium bromide, calcium iodide, calcium lactate, calcium carbonate, calcium phosphate, calcium citrate, calcium pyrophosphate, calcium glycerophosphate, calcium stearoyllactate, calcium pantothenate, calcium tartrate, calcium succinate, calcium malonate, calcium maleate, calcium nicotinate, calcium oxalate, calcium acetylsalicylate, calcium aluminosilicate, calcium borogluconate, calcium chlorate, calcium fluoride, calcium formate, calcium gluconate, calcium hypochlorite, calcium hypophosphite, calcium iodate, calcium levulinate, calcium magnesium acetate, calcium nitrite, calcium dioxide, calcium phenolsulfonate, calcium phosphite, calcium succinate, calcium sulfate, calcium sulfite, calcium thiocyanate, calcium thioglycollate, calcium glycerate, and calcium gluconate.

In practice calcium chloride and calcium acetate are the preferred calcium salts as they are cheap, widely available and have a good water solubility.

Typical examples of the lanthanum salt include lanthanum acetate, lanthanum oxalate, lanthanum citrate, lanthanum trifluoroacetate, lanthanum ethylhexanoate, lanthanum acetylacetonate, lanthanum nitrate, lanthanum chloride, lanthanum bromide, lanthanum iodide, lanthanum fluoride, lanthanum hydroxide, lanthanum sulphate, lanthanum carbonate or lanthanum phosphate.

In practice lanthanum chloride and lanthanum nitrate are the preferred lanthanum salts.

The quantity of the calcium salts as component (I) and the lanthanum salts as component (II) in the compositions according to the present invention will be so that a synergistic denaturing effect on allergens is obtained. As a general rule, the suitable proportions by weight of the amount of component (I) to component (II) is such that the ratio of the $Ca^{2+}$ ion over the $La^{3+}$ ion should lie in the range from 500:1 to 1:10 by weight. Other ranges are from 200:1 to 1:1, from 100:1 to 1:1, from 40:1 to 1:1, from 20:1 to 1:1, from 10:1 to 1:1, from 5:1 to 1:1 and from 2.5:1 to 1:1.

It is contemplated for the allergen denaturing compositions of the present invention that the amount of component (I) is present in a range from 0.1 % to 10% by weight of the $Ca^{2+}$ ion and the amount of component (II) is present in a range from 0.001 % to 5 % by weight of the $La^{3+}$ ion.

In a further embodiment the calcium salts (I) are present in a range from 0.5 % to 5 % by weight, or from 1% to 3%, by weight of the $Ca^{2+}$ ion and the lanthanum salts (II) are present in a range from 0.005 % to 0.1 %, or from 0.01 % to 0.05 %, by weight of the $La^{3+}$ ion of the allergen denaturing composition.

Depending upon the specific calcium salt or lanthanum salt used, the amount of said salt has to be calculated on the basis of the required amount of $Ca^{2+}$ ion or $La^{3+}$ ion that is needed.

The compositions according to the present invention comprise a combination of one or more calcium salts as component (I) and one or more lanthanum salts as component (II) wherein component (I) and component (II) are in respective proportions to produce a synergistic denaturing effect on allergens, and optionally one or more acceptable carriers.

These carriers are any material or substance with which the composition of components (I) and (II) is formulated in order to facilitate its application/dissemination to the locus to be treated, for instance by dissolving, dispersing, or diffusing the said composition, and/or to facilitate its storage, transport or handling without impairing its allergen denaturing effectiveness. Said acceptable carriers may be a solid or a liquid or a gas which has been compressed to form a liquid, i.e. the compositions of this invention can suitably be used as concentrates, emulsions, emulsifiable concentrates, encapsulations, oil miscible suspension concentrates, oil-miscible liquid, soluble concentrates, solutions, granulates, dusts, sprays, aerosols, pellets, or powders.

The allergen denaturing compositions of the present invention can be any formulation, such as a liquid, powder, paste and so on. Especially, liquid formulations are effective and easy to handle. One or more kinds of appropriate solvents, which are liquid carriers, that can be used for these formulations to dissolve or disperse the alkaline earth metal salts. The solvents are not restricted and for example, hydrophilic solvents such as water, methyl alcohol, ethyl alcohol, isopropyl alcohol, benzyl alcohol, acetic acid, acetone, dimethyl-formamide, dimethylacetamide, dimethyl sulfoxide, ethylene glycol, propylene glycol, diethylene glycol, triethylene glycol, dipropylene glycol, hexylene glycol, polyethylene glycol, glycerin, ethylene glycol monomethyl ether, ethylene glycol monoethyl ether, ethylene glycol monobutyl ether, ethylene glycol monoethyl ether acetate, gamma-butyrolactone, sulfolane and so on, hydrophobic solvents such as dimethyl-naphthalene, dodecylbenzene, liquid paraffin. isophorone, kerosene, dibutyl adipate, diethyl phthalate, diethylene glycol monobutyl ether acetate, propylene carbonate, palm oil, rapeseed oil, cottonseed oil, castor oil, soy bean oil and so on. Usually water, alcohols or their mixture are conveniently used as a suitable carrier.

Apart from both the aforementioned calcium salts (I) and lanthanum salts (II), the compositions according to the present invention may further comprise other known allergen denaturing or desactivating agents like tannic acid; modified cellulose, cedarwood oil, hexadecyltrimethylammonium chloride, aluminum chlorohydrate, 1- propoxy- propanol- 2, polyquaternium- 10, silica gel, propylene glycol alginate, ammonium sulphate, hinokitiol, L- ascorbic acid, chlorohexidine, immobilised tannic acid, maleic anhydride, hinoki oil, a composite of AgCl and TiO, diazolidinyl urea, 6- isopropyl- m- cresol, urea, cyclodextrin, hydrogenated hop oil, polyvinylpyrrolidone, N- methylpyrrolidone, the sodium salt of anthraquinone, potassium thioglycolate, and glutaraldehyde, dimethyl dialkyl ammonium salts, methyl trialkyl ammonium salts, tetraalkyl ammonium salts, tetramethyl ammonium salts, imidazoline based quaternaries, benzyl alkyl dimethyl ammonium salts, benzyl methyl dialkyl ammonium salts, benzyl trialkyl ammonium salts, dibenzyl dimethyl ammonium salts, propoxylated quaternary amines, ethoxylated quaternary amines, dialkyl diethoxylated ammonium salts, dipropoxylated ammonium salts, alkyl methyl diethoxylated ammonium salts, dipropoxylated ammonium salts, tetraethoxylated ammonium salts, tetrapropoxylated ammonium salts, methyl triethoxylated ammonium salts, propoxylated ammonium salts, ester quaternaries, phosphate- containing quaternary salts, pyridinium salts, polyvinyl pyridinium salts, vinyl pyridinium copolymer salts, nitrogen- containing heterocyclic amine salts, non- cellulose- based polyquaternium salts, vinyl pyrrolidone copolymers, dimethyl dialkyl ammonium salts, benzyl trialkyl ammonium salts, methyl trialkyl ammonium salts, tetraalkyl ammonium salts, tetramethyl ammonium salts, imidazoline- based quaternaries, benzyl alkyl dimethyl ammonium salts, benzyl methyl dialkyl ammonium salts, benzyl trialkyl ammonium salts, dibenzyl dimethyl ammonium salts, propoxylated quaternary amines, ethoxylated quaternary amines, dialkyl diethoxylated ammonium salts, dipropoxylated ammonium salts; methyl alkyl diethoxylated ammonium salts, methyl alkyl dipropoxylated ammonium salts, or benzyl alkyl diethoxylated ammonium salts, benzyl alkyl dipropoxylated ammonium salts, benzyl triethoxylated ammonium salts, benzyl tripropoxylated ammonium salts, methyl triethoxylated ammonium salts, methyl tripropoxylated ammonium salts, ester quaternaries, phosphate- containing quaternaries, pyridinium salts, polyvinyl pyridinium salts, vinyl pyridinium copolymer salts, nitrogen- containing heterocyclic amine salts, non- cellulose- based polyquaternium salts, sulfobetaines, betaines, amino carboxylates, amine oxides, amides, nitrogen- containing heterocyclic amides, dimethyl dialkyl ammonium salts, methyl trialkyl ammonium salts, tetraalkyl ammonium salts, tetramethyl ammonium salts, imidazoline- based quaternaries, benzyl alkyl dimethyl ammonium salts, benzyl methyl dialkyl ammonium salts, benzyl trialkyl ammonium salts, dibenzyl dimethyl ammonium salts, propoxylated quaternary amines, ethoxylated quaternary amines, dialkyl diethoxylated ammonium salts, dipropoxylated ammonium salts, alkyl methyl diethoxylated ammonium salts, dipropoxylated ammonium salts, tetraethoxylated ammonium salts, tetrapropoxylated ammonium salts, methyl triethoxylated ammonium salts, tripropoxylated ammonium salts, ester quaternarys, phosphate- containing quaternaries, pyridinium salts, polyvinyl pyridinium salts, vinyl pyridinium copolymer salts, nitrogen- containing heterocyclic amine salts, non- cellulose- based polyquaternium salts, amino carboxylates, amine oxides, amides, nitrogen- containing heterocyclic amides, vinyl pyrrolidone copolymers, aluminum sulfate, aluminum nitrate, aluminum chloride, magnesium sulfate, magnesium nitrate, magnesium chloride, cedarwood oil and mixtures thereof.

For the purpose of removing house dust mite allergens, it is effective to apply the present allergen denaturing compositions with acaricides or miticides. Miticides acceptable for use in the present invention include compounds known under the common names as pyrethroids like acrinathrin, allethrin, d-cis-trans allethrin, d-trans allethrin, bifenthrin, bioallethrin, bioallethrin S-cylclopentenyl, bioresmethrin, cycloprothrin, cyfluthrin, beta-cyfluthrin, cyhalothrin, lambda-cyhalothrin, gamma-cyhalothrin, cypermethrin, alpha-cypermethrin, beta-cypermethrin, thetacypermethrin, zeta-cypermethrin, cyphenothrin, deltamethrin, empenthrin, esfenvalerate, etofenprox, fenpropathrin, fenvalerate, flucythrinate, flumethrin, tau-fluvalinate, halfenprox, imiprothrin, permethrin, phenothrin, prallethrin, resmethrin, RU 15525, silafluofen, tefluthrin, tetramethrin, tetramethrin, tralomethrin, transfluthrin, ZXI 8901, lubrogthrinate, carboxylate; organic phosphorus compounds such as sumithion, fenthion, tetrachlorvinphos, malathion, diazinon and DDVP; or carbamates like carbaryl or propoxur, avermectins such as abamectin, emamectin benzoate or milbemectin, chlofentezin, flufenzine, hexythiazox, etoxazole, diafenthiuron, organotin miticides such as azocyclotin, cyhexatin and, fenbutatin oxide, amitraz, acequinocyl, bifenazate, dicofol, hydramethylnon, borax, sucrose esters like sucrose octanoate, fluacrypyrim, propargite, tetradifon, chlorfenapyr, benzoyl fenyl urea such as bistrifluron, chlofluazuron, diflubenzuron, flucycloxuron, flufenoxuron, hexaflumuron, lufenuron, novaluron, noviflumuron, teflubenzuron, triflumuron, METI-acaricides such as fenazaquin, fenpyroximate, pyrimidifen, pyridaben, tebufenpyrad, tolfenpyrad and tetronic acids such as spirodiclofen or spiromesofen.

A number of less toxic miticidal agents have been proposed for use in controlling dust mites. As noted in US- 4, 800, 196, these include phenyl salicylate, diphenylamine, methyl β- naphthyl ketone, coumarin, phenethyl benzoate, benzyl salicylate, phenyl benzoate, N- fluorodichloromethylthio- cyclohexene- dicarboxyimide, p- nitrobenzoic acid methyl ester, p- chlorometaxylenol, α- bromocinnamic aldehyde, 2, 5- dichloro- 4- bromo- phenol, N, N- dimethyl- N'- tryl- N'- (fluorodichloromethylthio)- sulfamide, 2- phenylphenol, sodium 2- phenylphenolate, 5- chloro- 2- methyl- 4- isothiazoline- 3- one, 2- methyl- 4- isothia- zonoline- 3- one and benzimidazolylmethyl- carbamate and mixtures of these. One of the more effective agents for killing dust mites is benzyl benzoate, a compound which is readily available and inexpensive. Acaricides can as well be derived from natural origins like carboxylic acid, formic acid, oxalic acid, thymol, geraniol, eugenol, pyrethrin, rotenone, azadirachtin, α- pinene, β- caryophyllene, myrcene, 1, 8- cineole, limonene, butylidenephtalide, monoterpenes like linalyl acetate, myrtenyl acetate, perillyl acetate and thymyl acetate, diterpenes like rediocides, flavanones like versuvanone, oxaporphine liriodenine. Furthermore, whole plant extracts containing essential oils can have miticidal activity (pinus tree oil, tea tree oil, eucalyptus oil, bergamot oil, grapefruit oil, clove oil, cinnamon oil, citronella oil, vetiver oil, neem tree oil, rose oil, eucalyptus oil, sandalwood oil, etc....) . Furthermore, most of these natural plant extracts have repellent or anti- feedant properties to the mites and to insects or could be used as a fragrance.

When one or more optional miticides are added to the composition of the present invention they are typically present at a level of from about 0.01% to about 20%, preferably from about 0.1 % to about 10%, more preferably from about 0.2% to about 8% by weight of the allergen denaturing composition.

Apart from both the aforementioned calcium salts (I) and lanthanum salts (II), the compositions according to the present invention may further comprise fungicides since some authors claim that the application of fungicides to a textile surface can kill micro- organisms necessary to degrade human skin dander into particles that can be ingested by the house dust mite. Indirectly, the dust mite population would starve and the number of house dust mite allergens would be reduced. Fungicides acceptable for use in the present invention include compounds known under the common names as 2- aminobutane; 2, 6- di- chloro- N- (4- trifluoromethylbenzyl) benzamide (XRD- 563) ; 8- hydroxyquinoline sulphate; 4- [3- (3, 4- dimethoxyphenyl)- 3- (4- fluorophenyl) acryloyl]- morpholine (SYP- L190) ; 2- (Thiocyanatomethylthio) benzo [d] thiazole (TCMTB) ; 4, 7- dimethyl- 1 H- indole- 2- carboxylic acid, methyl ester (OK- 9601) ; 1- (2, 6- dichlorophenyl)- 3, 4- dihydro- 6, 7- dimethoxy- isoquinoline (NSC- 338509) ; 1- [(4- chlorophenyl) methyl]- 4- phenyl-

EP 2 323 485 B1

piperidine dodecanoate (AC- 902202) ; cis- 1- (4- chlorophenyl)- 2- (1 H- 1, 2, 4- triazol- 1- yl)- cycloheptanol (SSF- 109) ; 6- bromo- 5- [(3, 5- dimethyl- 4- isoxazolyl) sulfonyl]- 2, 2- difluoro- 5H- 1, 3- dioxolo [4, 5- f] benzimidazole, 6- chloro- 5- [(3, 5- dimethyl- 4- isoxazolyly sulfonyl]- 2, 2- difluoro- 5H- 1, 3- dioxolo [4, 5- f] benzimidazole; 1- methoxy- 1H- indole- 3- carboxylic acid methyl ester; N- cyclohexylbenzo [b] thiophene- 2- carboxamide 1, 1- dioxide; (E)- O- 2- deoxy- 2- [(1- oxo- 9- octadecenyl) aminol- β- D- glucopyranosyl- (1→4)- O- 2- (acetyiamino)- 2- deoxy- β- D- glucopyranosyl- (1→4)- O- 2- (acetylamino)- 2- deoxy- β- D- glucopyranosyl- (1→4)- 2- (acetylamino)- 2- deoxy- D- glucose; (2S)- N- [2- [4- [[3- (4- chlorophenyl)- 2- propynyl] oxy]- 3- methoxyphenyl] ethyl]- 3- methyl- 2- [(methyl- sulfonyl)- amino] butanamide; (3- chloro- 6- hydroxy- 2- methylphenyl) (2, 3, 4- trimethoxy- 6- methylphenyl) methanone; 2, 4- dihydro- 5- methoxy- 2- methyl- 4- [2- [[[[1- [3- (trifluoro- methyl)- phenyl] ethylidene] amino] oxy] methyl] phenyl]- 3H- 1, 2, 4- triazol- 3- one; [(1S)- 1- [[[(1R)- 1- (6- fluoro- 2- benzothiazolyl) ethyl] amino] carbonyl]- 2- methylpropyl]- carbamic acid 1- methylethyl ester; 2, 6- dichloro- N- [[3- chloro- 5- (trifluoromethyl)- 2- pyridinyl]- methyl]- benzamide; N- (6- methoxy- 3- pyridinyl)- cyclopropanecarboxamide (ICIA- 0858) ; 1- (1, 2, 3, 4- tetrahydro- 1- naphthyl)- 5- (ethoxycarbonyl)- imidazole and its salts; 10- oxo- trans- 8- decenoic acid; 5- hydroxy- 2- hydroxymethyl- 4H- pyran- 4- one; 5- nitro- 1- indanone; phenylphenol (OPP), acibenzolar- S- methyl, aldimorph, ampropylfos, anilazine, benalaxyl, benodanil, benomyl, benthiavalicarb- isopropyl, binapacryl, biphenyl, bitertanol, blasticidin- S, borax, boscalid, bupirimate, buthiobate, sec- butylamine, caffeine, calcium polysulphide, captafol, captan, carbendazim, carboxin, carpropamid, carvacrol, quinomethionate, chloroneb, chloropicrin, chlorothalonil, chlozolinate, chromofungin, trans- cinnamaldehyde, trans- cinnamic acid, cinnamyl alcohol, cryptosporiopsin, cufraneb, cyazofamid, cyflufenamid, cymoxanil, cyprodinil, cyprofuram, dazomet, debacarb, dichlorphen, diclobutrazol, diclocymet, diclomezine, dichlofluanid, diclomezin, dicloran, diethofencarb, difenzoquat metilsulfate, diflumetorim, dimethirimol, dimethomorph, dinobuton, dinocap, diphenylamine, dipyrithion, ditalimfos, dithianon, dodemorph, dodine, drazoxolon, echinocandin, edifenphos, ethirimol, ethaboxam, etridiazole, eugenol, famoxadone, fenamidone, fenarimol, fenfuram, fenhexamid, fenitropan, fenoxanil, fenpiclonil, fenpropidin, fenpropimorph, fentin acetate, fentin hydroxide, ferbam, ferimzone, fluazinam, fludioxonil, flumetover, flumorlin, fluorofolpet, fluoromide, flusulfamide, flutolanil, flutriafol, flusulfamide, folpet, fosetyl- aluminium, fthalide, fuberidazole, furalaxyl, furmecyclox, griseofulvin, guazatine, harpin, hexachlorobenzene, hinokitiol, hydroxy- jesterone, hymexazol, imazalil, iminoctadine, iprobenfos (IBP), iprodione, iprovalicarb (fencaramid), isoprothiolane, jesterone, lanoconazole, kasugamycin, copper preparations such as: copper hydroxide, copper naphthenate, copper oxychloride, copper sulphate, copper oxide, oxine- copper and Bordeaux mixture, mancopper, mancozeb, maneb, mepanipyrim, mepronil, metalaxyl, methasulfocarb, methfuroxam, metiram, metrafenone, metsulfovax, myclobutanil, nabam, natamycin, nickel dimethyldithiocarbamate, nitrothal- isopropyl, nuarimol, octhilinone, ofurace, oxadixyl, oxamocarb, oxycarboxin, pefurazoate, pencycuron, pentalongin, phomalactone, phosdiphen, phthalide, pimaricin, piperalin, piroctone olamine, polyoxin, polyoxorim, probenazole, prochloraz, procymidone, propamocarb, propineb, propyl gallate, pseudomycin, pyrazophos, pyributicarb, pyrifenox, pyrimethanil, pyroquilon, pyrrolnitrin, quinoclamine, quinoxyfen, quintozene (PCNB), resveratrol, rustmicin. salicylanilide, silthiofam, spiroxamin, sulphur and sulphur preparations, tecloftalam, tecnazene, terpineol, terpinen- 4- ol, theophylline, thiabendazole, thicyofen, thiophanatemethyl, thiram, tiadinil (NNF- 9850), tolclophos- methyl, tolylfluanid, triazoxide, trichlamide, tricyclazole, tridemorph, triflumizole, triforine, umbelliferone, validamycin A, vinclozolin, zineb, ziram, zopfiellin, zosteric acid, zoxamide; isothia- and benzisothiazolone derivatives such as, e.g. 1, 2- benzisothiazolone (BIT), N- alkyl 1, 2- benzisothiazolones including butyl- BIT, DCOIT, OIT: zinc pyrithione, sodium pyrithione, oxypyrithione, fungicidal triazoles such as azaconazole, bitertanol, bromuconazole, cyproconazole, difenoconazole, diniconazole, epoxiconazole, fenarimol, fenbuconazole, fluquinconazole, flusilazole, flutriafol, hexaconazole, imibenconazole, ipconazole, ketoconazole, metconazole, myclobutanil, oxpoconazole, penconazole, propiconazole, prothioconazole, simeconazole, tebuconazole, tetraconazole, triadimefon, triadimenol, and triticonazole; fungicidal triazolopyrimidines like 5- chloro- 6- (2- chloro- 6- fluorophenyl)- 7- (4- methyl- 1- piperidinyl)- 2- [1, 2, 4] triazolo- [1, 5- a] pyrimidine; 5- chloro- N- (2, 2, 2- trifluoro- 1- methylethyl)- 6- (2, 4, 6- trifluorophenyl)- [1, 2, 4] triazolo [1, 5- a] pyrimidin- 7- amine; 5- chloro- N- (2, 2, 2- trifluoroethyl)- 6- (2, 4, 6- trifluorophenyl)- [1, 2, 4] triazolo [1, 5- a] pyrimidin- 7- amine; fungicidal thienopyrimidinones like 6- chloro- 2- propoxy- 3- propyl- thieno [2, 3- d] pyrimidin- 4 (3H)- one; strobilurines such as azoxystrobin, dimoxystrobin, enestroburin, fluoxastrobin, metominostrobin, orysastrobin, pyraclostrobin, kresoxim- methyl, trifloxystrobin, picoxystrobin, 2- [[[cyclopropyl[(4- methoxyphenyl) imino] methyl] thio] methyl]- α- (methoxymethylene)- benzeneacetic acid methyl ester (UBF- 307) and (E, E)- α- (methoxyimino)- N- methyl- 2- [[[1- [3- (trifluoromethyl) phenyl] ethoxy] imino] methyl]- benzeneacetamide (MA- 565) .

The present allergen denaturing compositions can also contain a surfactant, chelating agent, anti-oxidants, colorant, anticorrosive agent, preservatives, binder, thickener, anti-scale agents, antifoaming agents, antistatic agents, softener and so on. Further, the compositions may also comprise a water-soluble polymer selected from starch, polyvinyl alcohols, methyl cellulose and its derivatives, polyacrylic acids, polyethylene glycol, polyquaternium compounds, polyvinyl pyrrolidone (PVP) and its derivatives and mixtures thereof.

When the compositions of the present invention are used as a spray product an optional fragance can be included to provide a pleasing scent. Frangances are typically added at low levels, e.g., from about 0.01% to about 0.05% by weight of the composition. Any type of frangance can be used. Typical frangances are e.g. allyl caproate, amyl

5

acetate, amyl propionate, anisic aldehyde, anisole, benzaldehyde, benzyl acetate, benzyl acetone, benzyl alcohol, benzyl formate, benzyl iso valerat, benzyl propionate, beta gamma hexenol, camphor gum, laevo- carveol, d- carvone, laevo-carvone, cinnamyl formate, cis- jasmone, cis- 3- hexenyl acetate, cuminic alcohol, cuminic aldehyde, cyclal C, dimethyl benzyl carbinol, dimethyl benzyl carbinyl acetate, ethyl acetate, ethyl aceto acetate, ethyl amyl ketone, ethyl benzoate, ethyl butyrate, ethyl hexyl ketone, ethyl- 2- methyl butyrate, ethyl methyl pentanoate, ethyl phenyl acetate, eucalyptol, fenchyl alcohol, flor acetate (tricyclo decenyl acetate), frutene (tricyclo decenyl propionate), geraniol, hexenol, hexenyl acetate, hexyl acetate, hexyl formate, hydratropic alcohol, hydroxycitronellal, isoamyl alcohol, isomenthone, isopulegyl acetate, tsoquinoline, ligustral, linalool, linalool oxide, linalyl formate, menthone, methyl acetophenone, methyl amyl ketone, methyl anthranilate, methyl benzoate, methyl benzyl acetate, methyl eugenol, methyl heptenone, methyl heptine carbonate, methyl heptyl ketone, methyl hexyl ketone, methyl phenyl carbinyl acetate, methyl salicylate, nerol, octalactone, octyl alcohol (octanol- 2), para- cresol, para- cresyl methyl ether, para- methyl acetophenone, phenoxy ethanol, phenyl acetaldehyde, phenyl ethyl acetate, phenyl ethyl alcohol, phenyl ethyl dimethyl carbinol, prenyl acetate, propyl butyrate, pulegone, rose oxide, safrole, 4- terpinenol, allyl heptoate, anethol, benzyl butyrate camphene, carvacrol, cis-3- hexenyl tiglate, citral, citronellol, citronellyl acetate, citronellyl isobutyrate, citronellyl nitrile, citronellyl propionate, cyclohexyl ethyl acetate, decyl aldehyde, delta damascone, dihydro myrcenol, dihydromyrcenylacetate, dimethyl octanol, fenchyl acetate, gamma methyl lonone, gamma- nonalactone, geranyl acetate, geranyl formate, geranyllsobutyrate, geranyl nitrile, hexenyllsobutyrate, hexyl neopentanoate, hexyl tiglate, alpha- lonone, beta- lonone, gamma- lonone, alpha- irone, isobornyl acetate, isobutyl benzoate, isononyl acetate, isononyl alcohol, isomenthol, para- isopropyl phenylacetaldehyde, isopulegol lauric aldehyde (dodecanal), d- limonene, linalyl acetate, menthyl acetate, methyl chavicol, alpha- iso"gamma"methyl ionone, methyl nonyl acetaldehyde, methyl octyl acetaldehyde, myrcene, neral, neryl acetate, nonyl acetate, nonyl aldehyde, octyl aldehyde, orange terpenes, para- cymene, phenyl ethyl isobutyrate, alpha- pinene, beta- pinene, alpha- terpinene, gamma- terpinene, terpinolene, terpinyl acetate, tetrahydro linalool, tetrahydro myrcenol, undecenal, veratrol, verdox, vertenexalpha allyl cyclohexane propionate, ambrettolide, amyl benzoate, amyl cinnamate, amyl cinnamic aldehyde, amyl cinnamic aldehyde dimethyl acetal, iso- amyl salicylate, aurantiol, benzophenone, benzyl salicylate, cadinene, cedrol, cedryl acetate, cinnamyl cinnamate, coumarin, cyclohexyl salicylate, cyclamen aldehyde, dihydro isojasmonate, diphenyl methane, ethylene brassylate, ethyl methyl phenyl glycidate, ethyl undecylenate, iso-eugenol, exaltolide, galaxolide, geranyl anthranilate, hexadecanolide, hexenyl salicylate, hexyl cinnamic aldehyde, hexyl salicylate, linalyl benzoate, 2- methoxy naphthalene, methyl cinnamate, methyl dihydrojasmonate, beta- methyl naphthyl ketone, musk indanone, musk ketone, musk tibetine, myristicin, delta- nonalactone, oxahexadecanolide- 10, oxahexadecanolide- 11, patchouli alcohol, phantolide, phenyl ethyl benzoate, phenylethylphenylacetate, alpha- santalol, thibetolide, delta- undecalactone, gamma- undecalactone, vanillin, vetiveryl acetate, yara- yara and mixtures thereof.

The compositions of the present invention when used in liquid form, such as a spray product, may further comprise an antimicrobial preservative. The antimicrobial preservative is present in amount that is effective to prevent spoilage of the allergen denaturing composition in order to increase its shelf-life. Preferred levels of preservative are from about 0.0001% to about 0.5%, more preferably from about 0.0002% to about 0.2% by weight of the composition. The preservative can be any organic preservative material which will not cause damage to fabric appearance, e.g., discoloration, coloration, bleaching. Preferred water-soluble preservatives include organic sulfur compounds, halogenated compounds, cyclic organic nitrogen compounds, low molecular weight aldehydes, quaternary ammonium compounds, dehydroacetic acid, phenyl and phenolic compounds, and mixtures thereof.

When the allergen denaturing compositions of the present invention are used as a spray product they are generally used for directly spraying onto the carpets, tatami mats, bedclothes, matrasses, pet pillows, curtains, air filters, stuffed animals and other materials that are contaminated by allergens using a spray dispenser. When the allergen denaturing composition of the present invention are used as a pre-treatment of carpets, tatami mats, bedclothes, matrasses, pet pillows, curtains, air filters, stuffed animals and other materials before marketing to serve as a protection against allergens which might be in contact with these materials during later use.

The allergen denaturating compositions of the present invention may also be combined with products or compounds bringing cleaning, cleansing or odour removing properties in the formulation.

In another embodiment an *ex vivo* method for denaturing allergens is claimed which method comprises applying an effective amount of an allergen denaturing composition of the present invention to the place where allergens exist. Furthermore the allergen denaturing compositions can also be used in an *ex vivo* method for denaturing allergens which method comprises pre-treatment of materials with an effective amount of said allergen denaturing composition to reduce the presence of allergens in the later use of the materials.

The term "effective amount" of an allergen denaturing composition in accordance with the presence invention, means that amount of composition comprising a combination of one or more calcium salts as component (I) and one or more lanthanum salts as component (II) that neutralize at least about 50%, preferably at least about 60%, more preferably at least about 80%, and most preferably at least about 90% of the allergens on the surface or article that is treated. The amount of allergen that is neutralized can be measured by the ELISA test described below. The effective amount can be determined using routine optimization techniques and is dependent upon the particular allergen and material to be treated, the route of administration, the formulation, and other factors evident to those skilled in the art. An effective amount may be achieved by multiple dosing, by combining component (I) and (II) in one combination product, or by application of component (I) and (II) separately in a short sequence or by application of component (I) on a surface already containing component (II) or by application of component (II) on a surface already containing component (I).

**Experimental Part**

**Typical formulation examples**

|  | Formulation (1) | Formulation (2) | Formulation (3) |
|---|---|---|---|
| $CaCl_2.2H_2O$ | 33 g/kg | 66 g/kg | 66 g/kg |
| $LaCl_3.7H_2O$ | 0.260 g/kg | 0.650 g/kg | 1.3 g/kg |
| Calcium acetate monohydrate | - | 7.4 g/kg | 7.4g/kg |
| polyacrylic acid | - | 10 g/kg | - |
| lauryl glycol ether | 7.5 g/kg | - | 7.5 g/kg |
| Benzalkonium chloride | 1 g/kg | 1 g/kg | 1 g/kg |
| polyethylene glycol | - | 10 g/kg | - |
| ethanol | - | 150 g/kg | - |
| Water | up to 1 kg | up to 1 kg | up to 1 kg |

**Experiment 1 : Effects of $Ca^{2+}/La^{3+}$ on Der p1**

Allergen stock solutions were derived from the extraction of the rearing medium of dust mites *Dermatophagoides pteronyssinus* or by the extraction of cat exposed tissues. Allergens were always stored or diluted in Phosphate Buffered Saline (PBS) of pH 7.4 containing NaCl 8g/L, $KH_2PO_4$ 0.2 g/L, $Na_2HPO_4$ 1.15 g/L, KCl 0.2 g/L, Tween 20 0.5 ml/L.

A tissue (Triconet : 40 % PP, 60 % Viscose, 0.7 mm thickness, 70 g/m$^2$) was treated with known amount of antigen, allowed to dry overnight and then treated with the allergen denaturing composition at 100 ml/m$^2$. The next day, the tissue was extracted in 5 ml of PBS and the concentration of allergen was determined by ELISA. ELISA tests were carried out according to the instructions of the commercially available test kits with monoclonal antibodies (www.inbio.com).

Briefly, anti- Der p1 or anti- Fel d1 monoclonal antibodies are diluted 1/1000 in 50 mM carbonate- bicarbonate buffer, pH 9.6. and 100 $\mu$l is coated on a polystyrene microtiter well and allowed to incubate overnight at 4°C. Then, wells are washed 3 times with PBS containing 0.05 % Tween 20, pH 7.4. (further called : wash step with PBS- T) . After incubation with 100 $\mu$l 1 % BSA in PBS- T for 30 minutes at room temperature, another wash step is executed. Then, 100 $\mu$l of diluted allergen standard or test samples is added to each well and incubated for 1 hour at room temperature. After a wash step, 100 $\mu$l per well of 1/1000 diluted biotinylated anti- Der p1 or anti- Fel d1 monoclonal antibody is added and incubated for 1 hour at room temperature. Then, another wash step is executed and 100 $\mu$l/ well of a Streptavidin- Peroxidase (Sigma S5512, reconstituted in 1 ml distilled water) is added as a 1/1000 dilution in PBS- T and incubated for 30 minutes at room temperature. After a final wash step, 100 $\mu$l/ well of 1 mM ABTS (2, 2'- azino- bis (3- ethylbenzthiazoline- 6- sulfonic acid) diammonium salt) in 70 mM citrate phosphate buffer, pH 4.2, containing 0.03 % $H_2O_2$, is added. The assay was allowed to develop after dark green colouring of the highest concentration of the allergen standard. Optical density was read at 405 nm.

Standard allergen dilution curves were taken for calibration. The range in which allergens can be quantified is 0.2-20 ppb for Fel d1 and from 1-100 ppb for Der p1. Dilutions were made until the concentration was in the right order of magnitude to enable quantification and percent reduction by the allergen denaturing composition..

Table 1 : efficacy of $Ca^{2+}$ on Der p1; or $La^{3+}$ on Der p1

| $Ca^{2+}$ (%) | Efficacy (%) |  | $La^{3+}$ (%) | Efficacy (%) |
|---|---|---|---|---|
| 0.05 | 0 |  | 0 | 0 |
| 0.125 | 0 |  | 0.01 | 12 |
| 0.5 | 28 |  | 0.05 | 0 |
| 2.0 | 96 |  | 0.2 | 0 |

(continued)

| Ca$^{2+}$ (%) | Efficacy (%) | | La$^{3+}$ (%) | Efficacy (%) |
|---|---|---|---|---|
| | | | 1.0 | 100 |

Possible synergy was investigated using Limpel's formula (Richter, D.L., Pestic. Sci. 1987, 19: 309-315):

$$E_c = X + Y - [(X.Y)/100]$$

where $E_c$ is the expected additive response, or calculated activity, X is the observed percentage control when component A is applied alone and Y is the observed percentage control when component B is applied alone. Synergy was considered to occur when the observed effect, or measured activity, of a combination of both components was greater than the corresponding $E_c$ value.

Table 2 : efficacy of Ca$^{2+}$/La$^{3+}$ on Der p1

| | Ca$^{2+}$ (1%) | | |
|---|---|---|---|
| | Efficacy (%) | | |
| La$^{3+}$ (%) | Expected value ($E_c$) | Measured value | Synergy |
| 0 | 46 | 46 | 0 |
| 0.010 | 46 | 67 | 21 |
| 0.025 | 46 | 89 | 43 |
| 0.050 | 46 | 97 | 51 |

## Experiment 2 : synergy test on 96 well plate

Allergen denaturing activity against the cat allergen Fel d1 was determined by the addition of an identical aliquot of a diluted natural stock solution of Fel d1 to each well of a 96 well plate. Allergen stocks were prepared as mentioned in the description of experiment 1. Every row of a 96 well plate contained a serial dilution of one of the following : calcium, lanthanum salts or a combination of both salts.

After incubation of each plate for 1 hour at room temperature under gentle agitation, a 100 μl sample was taken for analysis with ELISA as described in experiment 1.

The EC90 was determined as the concentration of allergen denaturing compounds that reduced the allergen concentration by 90 % compared to the wells in which only the allergens were added to a PBS solution. Synergy was calculated using the Synergy Index method (Kull *et al.* 1961, Lada et al. 1977, Steinberg 2000, Zwart Voorspuij & Nass 1957).

$$\text{Synergy Index (SI)} = (x/m) + (y/n)$$

where:

- m and n are the EC90 values of compounds 1 and 2 respectively, when applied alone,
- x and y are the respective quantities of compounds 1 and 2 in mixtures which also cause the 90 % inhibition of allergens, *i.e*. x and y equal [fraction of compound 1 and 2, resp., in the mixture (e.g. 0.8 and 0.2, 066 and 0.33...)] multiplied by [EC90-value of the mixture].

When the Synergy Index is greater than 1.0, antagonism is indicated. When the SI is equal to 1.0, additivity is indicated. When the SI is less than 1.0, synergism is demonstrated.

Table 3 : efficacy of calcium and lanthanum on Fel d1 allergen

| $Ca^{2+}/La^{3+}$ ratio | ppm $Ca^{2+}$ | ppm $La^{3+}$ | EC90 | Synergy Index |
|---|---|---|---|---|
| - | 100 | 0 | 1250 | - |
| 20 | 95.2 | 4.8 | 630 | 0.95 |
| 10 | 90.9 | 9.1 | 231 | 0.50 |
| 5 | 83.3 | 16.7 | 142.38 | 0.47 |
| 2.5 | 71.4 | 28.6 | 110.73 | 0.56 |
| 1.25 | 55.6 | 44.4 | 64.07 | 0.48 |
| - | 0 | 100.0 | 63.26 | - |

**Claims**

1. An *ex vivo* method for denaturing allergens which comprises applying an effective amount of a composition comprising a combination of one or more calcium salts as component (I) and one or more lanthanum salts as component (II) wherein component (I) and component (II) are in respective proportions to produce a synergistic denaturing effect on allergens to the place where allergens exist.

2. The method as claimed in claim 1 wherein the one or more calcium salts as component (I) are selected from calcium acetate, calcium propionate, calcium nitrate, calcium chloride, calcium bromide, calcium iodide, calcium lactate, calcium carbonate, calcium phosphate, calcium citrate, calcium pyrophosphate, calcium glycerophosphate, calcium stearoyllactate, calcium pantothenate, calcium tartrate, calcium succinate, calcium malonate, calcium malate, calcium oxalate, calcium acetylsalicylate, calcium aluminosilicate, calcium borogluconate, calcium chlorate, calcium fluoride, calcium formate, calcium gluconate, calcium hypochlorite, calcium hypophosphite, calcium iodate, calcium levulinate, calcium magnesium acetate, calcium nitrite, calcium dioxide, calcium phenolsulfonate, calcium phosphite, calcium succinate, calcium sulfate, calcium sulfite, calcium thiocyanate, calcium thioglycollate, calcium nicotinate, calcium glycerate, and calcium gluconate.

3. The method as claimed in claim 1 or 2 wherein the one or more lanthanum salts as component (II) are selected from lanthanum acetate, lanthanum nitrate, lanthanum chloride, lanthanum bromide, lanthanum iodide, lanthanum carbonate? lanthanum oxalate, lanthanum citrate, lanthanum trifluoroacetate, lanthanum ethylhexanoate, lanthanum acetylacetonate, lanthanum fluoride, lanthanum hydroxide, lanthanum sulphate, or lanthanum phosphate.

4. The method as claimed in claim 3 wherein the calcium salt (I) is selected from calcium chloride and calcium acetate and the lanthanum salt (II) is selected from lanthanum chloride or lanthanum nitrate.

5. The method according to any of claims 1 to 4 wherein the ratio of the $Ca^{2+}$ ion over the $La^{3+}$ ion is in the range from 500:1 to 1:10 by weight.

6. The method according to claim 5 wherein the ratio of the $Ca^{2+}$ ion over the $La^{3+}$ ion is in the range from 100:1 to 1:1 by weight.

7. The method according to any of claims 1 to 4 wherein the amount of component (I) is present in a range from 0.1 % to 10% by weight of $Ca^{2+}$ and the amount of component (II) is present in a range from 0.001 % to 5 % by weight of $La^{3+}$.

8. The method according to claim 7 wherein the amount of component (I) is present in a range from 0.5 % to 5 % by weight of $Ca^{2+}$ and the amount of component (II) is present in a range from 0.005 % to 0.1 % by weight of $La^{3+}$.

9. The method according to claim 8 wherein the amount of component (I) is present in a range from 1 % to 3 % by weight of $Ca^{2+}$ and the amount of component (II) is present in a range from 0.01 % to 0.05 % by weight of $La^{3+}$.

10. A method for denaturing allergens which comprises pre-treatment of materials with an effective amount of an allergen denaturing composition as described in any of claims 1 to 9 to reduce the presence of allergens in their later use.

**Patentansprüche**

1. Ex-vivo-Verfahren zum Denaturieren von Allergenen, bei dem man eine wirksame Menge einer Zusammensetzung, die eine Kombination von einem oder mehreren Calciumsalzen als Komponente (I) und einem oder mehreren Lanthansalzen als Komponente (II) appliziert, wobei Komponente (I) und (II) in solchen Verhältnissen vorliegen, dass eine synergistische denaturierende Wirkung auf Allergene auf den Ort, wo Allergene vorliegen, ausgeübt wird.

2. Verfahren nach Anspruch 1, wobei das eine oder die mehreren Calciumsalze als Komponente (I) aus der Reihe Calciumacetat, Calciumpropionat, Calcium-nitrat, Calciumchlorid, Calciumbromid, Calciumiodid, Calciumlactat, Calciumcarbonat, Calcium-phosphat, Calciumcitrat, Calciumpyrophosphat, Calciumglycerophosphat, Calciumstearoyllactat, Calciumpantothenat, Calciumtartrat, Calciumsuccinat, Calciummalonat, Calciummalat, Calciumoxalat, Calciumacetylsalicylat, Calciumaluminosilicat, Calciumborgluconat, Calciumchlorat, Calciumfluorid, Calciumformiat, Calciumgluconat, Calciumhypochlorit, Calciumhypophosphit, Calciumiodat, Calciumlevulinat, Calciummagnesiumacetat, Calciumnitrit, Calciumdioxid, Calciumphenolsulfonat, Calciumphosphit, Calciumsuccinat, Calciumsulfat, Calciumsulfit, Calciumthiocyanat, Calciumthioglycolat, Calciumnicotinat, Calciumglycerat und Calciumgluconat ausgewählt sind.

3. Verfahren nach Anspruch 1 oder 2, wobei das eine oder die mehreren Lanthansalze als Komponente (II) aus der Reihe Lanthanacetat, Lanthannitrat, Lanthanchlorid, Lanthanbromid, Lanthaniodid, Lanthancarbonat, Lanthanoxalat, Lanthancitrat, Lanthantrifluoracetat, Lanthanethylhexanoat, Lanthanacetylacetonat, Lanthanfluorid, Lanthanhydroxid, Lanthansulfat oder Lanthanphosphat ausgewählt sind.

4. Verfahren nach Anspruch 3, wobei das Calciumsalz (I) aus der Reihe Calciumchlorid und Calciumacetat ausgewählt ist und das Lanthansalz (II) aus der Reihe Lanthanchlorid oder Lanthannitrat ausgewählt ist.

5. Verfahren nach einem der Ansprüche 1 bis 4, wobei das Verhältnis des $Ca^{2+}$-Ions zu dem $La^{3+}$-Ion im Bereich von 500:1 bis 1:10 in Bezug auf das Gewicht beträgt.

6. Verfahren nach Anspruch 5, wobei das Verhältnis des $Ca^{2+}$-Ions zu dem $La^{3+}$-Ion im Bereich von 100:1 bis 1:1 in Bezug auf das Gewicht beträgt.

7. Verfahren nach einem der Ansprüche 1 bis 4, wobei die Menge an Komponente (I) in einem Bereich von 0,1 Gew.-% bis 10 Gew.-% $Ca^{2+}$ vorliegt und die Menge an Komponente (II) im Bereich von 0,001 Gew.-% bis 5 Gew.-% $La^{3+}$ vorliegt.

8. Verfahren nach Anspruch 7, wobei die Menge an Komponente (I) in einem Bereich von 0,5 Gew.-% bis 5 Gew.-% $Ca^{2+}$ vorliegt und die Menge an Komponente (II) im Bereich von 0,005 Gew.-% bis 0,1 Gew.-% $La^{3+}$ vorliegt.

9. Verfahren nach Anspruch 8, wobei die Menge an Komponente (I) in einem Bereich von 1 Gew.-% bis 3 Gew.-% $Ca^{2+}$ vorliegt und die Menge an Komponente (II) im Bereich von 0,01 Gew.-% bis 0,05 Gew.-% $La^{3+}$ vorliegt.

10. Verfahren zum Denaturieren von Allergenen, das die Vorbehandlung von Materialien mit einer wirksamen Menge einer allergendenaturierenden Zusammensetzung wie in einem der Ansprüche 1 bis 9 beschrieben umfasst, um das Vorhandensein von Allergenen bei deren weiterer Verwendung zu reduzieren.

**Revendications**

1. Méthode *ex vivo* de dénaturation d'allergènes, comprenant l'application d'une quantité efficace d'une composition comprenant une combinaison d'un ou plusieurs sels de calcium comme composant (I) et d'un ou plusieurs sels de lanthane comme composant (II), où le composant (I) et le composant (II) sont en proportions respectives afin de produire un effet dénaturant synergique sur des allergènes à l'endroit où se trouvent les allergènes.

2. Méthode selon la revendication 1, dans laquelle les un ou plusieurs sels de calcium comme composant (I) sont choisis parmi l'acétate de calcium, le propionate de calcium, le nitrate de calcium, le chlorure de calcium, le bromure de calcium, l'iodure de calcium, le lactate de calcium, le carbonate de calcium, le phosphate de calcium, le citrate de calcium, le pyrophosphate de calcium, le glycérophosphate de calcium, le stéaroyl-lactate de calcium, le pantothénate de calcium, le tartrate de calcium, le succinate de calcium, le malonate de calcium, le malate de calcium, l'oxalate de calcium, l'acétylsalicylate de calcium, l'aluminosilicate de calcium, le borogluconate de calcium, le chlorate de calcium, le fluorure de calcium, le formiate de calcium, le gluconate de calcium, l'hypochlorite de calcium, l'hypophosphite de calcium, l'iodate de calcium, le lévulinate de calcium, l'acétate de magnésium et de calcium, le nitrite de calcium, le dioxyde de calcium, le phénolsulfonate de calcium, le phosphite de calcium, le succinate de calcium, le sulfate de calcium, le sulfite de calcium, le thiocyanate de calcium, le thioglycolate de calcium, le nicotinate de calcium, le glycérate de calcium, et le gluconate de calcium.

3. Méthode selon la revendication 1 ou 2, dans laquelle les un ou plusieurs sels de lanthane comme composant (II) sont choisis parmi l'acétate de lanthane, le nitrate de lanthane, le chlorure de lanthane, le bromure de lanthane, l'iodure de lanthane, le carbonate de lanthane, l'oxalate de lanthane, le citrate de lanthane, le trifluoroacétate de lanthane, l'éthylhexanoate de lanthane, l'acétylacétonate de lanthane, le fluorure de lanthane, l'hydroxyde de lanthane, le sulfate de lanthane, ou le phosphate de lanthane.

4. Méthode selon la revendication 3, dans laquelle le sel de calcium (I) est choisi parmi le chlorure de calcium et l'acétate de calcium et le sel de lanthane (II) est choisi parmi le chlorure de lanthane ou le nitrate de lanthane.

5. Méthode selon l'une quelconque des revendications 1 à 4, dans laquelle le rapport des ions $Ca^{2+}$ aux ions $La^{3+}$ se trouve dans la plage allant de 500:1 à 1:10 en poids.

6. Méthode selon la revendication 5, dans laquelle le rapport des ions $Ca^{2+}$ aux ions $La^{3+}$ se trouve dans la plage allant de 100:1 à 1:1 en poids.

7. Méthode selon l'une quelconque des revendications 1 à 4, dans laquelle la quantité de composant (I) est présente dans la plage allant de 0,1% à 10% en poids de $Ca^{2+}$ et la quantité de composant (II) est présente dans la plage allant de 0,001% à 5% en poids de $La^{3+}$.

8. Méthode selon la revendication 7, dans laquelle la quantité de composant (I) est présente dans la plage allant de 0,5% à 5% en poids de $Ca^{2+}$ et la quantité de composant (II) est présente dans la plage allant de 0,005% à 0,1% en poids de $La^{3+}$.

9. Méthode selon la revendication 8, dans laquelle la quantité de composant (I) est présente dans la plage allant de 1% à 3% en poids de $Ca^{2+}$ et la quantité de composant (II) est présente dans la plage allant de 0,01% à 0,05% en poids de $La^{3+}$.

10. Méthode de dénaturation d'allergènes, comprenant le prétraitement de matériaux par une quantité efficace d'une composition de dénaturation d'allergènes telle que décrite selon l'une quelconque des revendications 1 à 9 afin de réduire la présence d'allergènes, lors de leur utilisation ultérieure.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 1133918 A
- EP 1131999 A

- US 4800196 A

**Non-patent literature cited in the description**

- **RICHTER, D.L.** *Pestic. Sci.,* 1987, vol. 19, 309-315